(19) **Europäisches Patentamt**
**European Patent Office**
**Office européen des brevets**

(11) **EP 4 768 905 A1**

(12) **EUROPEAN PATENT APPLICATION**

(43) Date of publication:
**01.07.2026 Bulletin 2026/27**

(21) Application number: **25224888.5**

(22) Date of filing: **18.12.2025**

(51) International Patent Classification (IPC):
**G01N 29/02** (2006.01)    **G01N 29/036** (2006.01)
**G01N 29/24** (2006.01)

(52) Cooperative Patent Classification (CPC):
**G01N 29/022; G01N 29/036; G01N 29/2437;**
G01N 2291/02433; G01N 2291/0256;
G01N 2291/02809

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **26.12.2024 JP 2024231231**

(71) Applicant: **Yokogawa Electric Corporation
Musashino-shi, Tokyo 180-8750 (JP)**

(72) Inventor: **SEKIMORI, Yukimitsu
Musashino-shi, Tokyo, 180-8750 (JP)**

(74) Representative: **Winter, Brandl - Partnerschaft
mbB
Alois-Steinecker-Straße 22
85354 Freising (DE)**

(54) **CHEMICAL SENSOR AND MEASUREMENT APPARATUS**

(57)    A chemical sensor 1 includes a silicon substrate 2 on which a diaphragm 21 is formed, a strain sensor 3 that is provided on the silicon substrate 2, an adsorption membrane 4 that is provided at the diaphragm 21 and whose volume changes by adsorbing a specific substance. The strain sensor 3 includes a vacuum chamber that is formed in the diaphragm 21, and a resonator that is formed in an interior of the vacuum chamber.

FIG.1

**Description**

CROSS-REFERENCE TO RELATED APPLICATION(S)

The present application claims priority to and incorporates by reference the entire contents of Japanese Patent Application No. 2024-231231 filed in Japan on December 26, 2024.

BACKGROUND OF THE INVENTION

1. Field of the Invention

The present disclosure relates to a chemical sensor and a measurement apparatus.

2. Description of the Related Art

There is a chemical sensor that measures a concentration of a specific substance included in a measuring medium by using an adsorption membrane that adsorbs the specific substance. For example, in Japanese Laid-open Patent Publication No. 2020-19102, a chemical sensor that is constituted by forming a resonator having a lead zirconate titanate (PZT) vibrating element structure on a silicon substrate, and directly forming an adsorption membrane at the resonator has been disclosed. In the chemical sensor disclosed in Japanese Laid-open Patent Publication No. 2020-19102, a concentration of the specific substance is calculated by measuring a change in resonance frequency of the resonator based on a mass change of the adsorption membrane that has adsorbed the specific substance.

However, in the chemical sensor described above, the adsorption membrane is directly formed at the resonator, so that the resonator comes into contact with the measuring medium. As a result of this, a change in state of the measuring medium have a significant effect on a change in the resonance frequency of the resonator, so that measurement accuracy of the concentration may sometimes decrease.

It is an object of the present disclosure to obtain a chemical sensor that is able to stably and accurately measures a concentration of a specific substance included in a measuring medium.

SUMMARY OF THE INVENTION

According to an aspect of an embodiment, a chemical sensor includes a silicon substrate on which a diaphragm is formed, a strain sensor that is provided on the silicon substrate, an adsorption membrane that is provided at the diaphragm and whose volume changes by adsorbing a specific substance. The strain sensor includes a vacuum chamber that is formed in the diaphragm, and a resonator that is formed in an interior of the vacuum chamber.

BRIEF DESCRIPTION OF THE DRAWINGS

FIG. 1 is a plan view of a chemical sensor according to a first embodiment;
FIG. 2 is a cross-sectional view of the chemical sensor taken along line II-II illustrated in FIG. 1;
FIG. 3 is a partial enlarged cross-sectional view of a portion A illustrated in FIG. 2 in an enlarged manner;
FIG. 4 is a plan view of a chemical sensor according to a first modification;
FIG. 5 is a cross-sectional view of the chemical sensor taken along line V-V illustrated in FIG. 4;
FIG. 6 is a cross-sectional view of a chemical sensor according to a second modification;
FIG. 7 is a cross-sectional view illustrating a schematic configuration of a measurement apparatus on which the chemical sensor according to the first embodiment is mounted;
FIG. 8 is a flowchart illustrating a measurement procedure of measuring a hydrogen concentration performed by using a single unit of a measurement apparatus; and
FIG. 9 is a flowchart illustrating a measurement procedure of measuring a hydrogen concentration performed by using two units of the measurement apparatuses.

DETAILED DESCRIPTION OF THE PREFERRED EMBODIMENTS

Hereinafter, preferred embodiments of a chemical sensor and a measurement apparatus disclosed in the present application will be explained in detail below with reference to the accompanying drawings. Furthermore, the present invention is not limited to the embodiments described below.

First Embodiment

Schematic configuration of chemical sensor

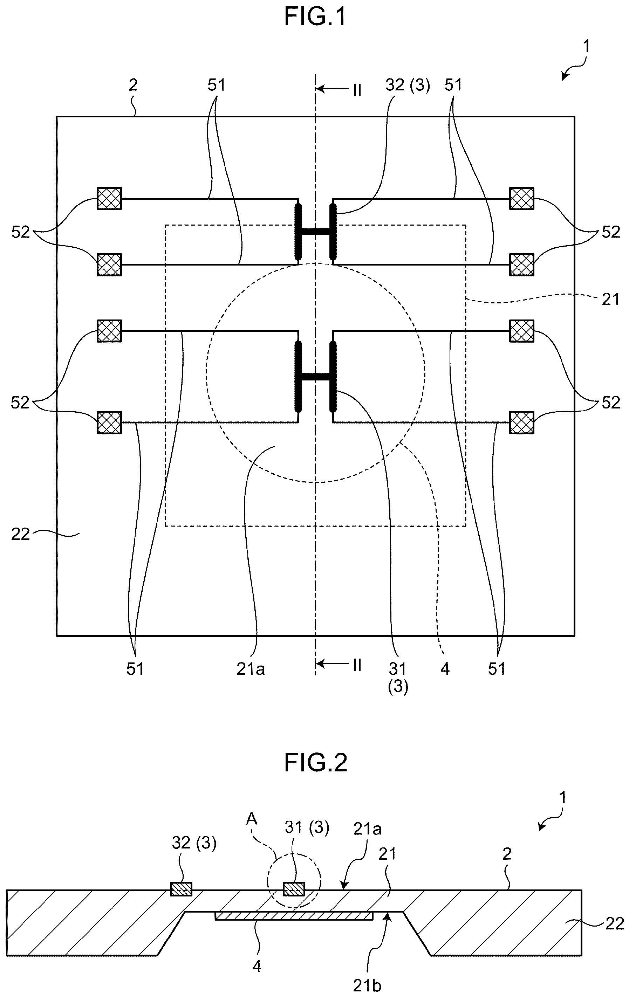

FIG. 1 is a plan view of a chemical sensor according to a first embodiment. FIG. 2 is a cross-sectional view of the chemical sensor taken along line II-II illustrated in FIG. 1. A chemical sensor 1 includes a silicon substrate 2, a strain sensor 3, and an adsorption membrane 4.

Regarding silicon substrate

The silicon substrate 2 is made of silicon. The silicon substrate 2 includes a diaphragm 21 and a rim portion 22. The diaphragm 21 is formed into a thin membrane. The diaphragm 21 includes a first surface 21a, and a second surface 21b that corresponds to the rear surface of the first surface 21a. The diaphragm 21 becomes deformed when a pressure is applied to the first surface 21a or the second surface 21b, or when a volume of the adsorption membrane 4 that will be described later is changed.
The rim portion 22 is formed so as to surround the circumference of the diaphragm 21 in a plan view. The rim portion 22 is formed to have a thickness that is thicker than that of the diaphragm 21.

Regarding of strain sensor

The strain sensor 3 is provided on the first surface 21a side of the silicon substrate 2. As the strain sensor 3, a first strain sensor 31 that is provided at the position at which the first strain sensor 31 overlaps with the diaphragm 21 in a plan view illustrated in FIG. 1, and a second strain sensor 32 that is provided at a boundary between the diaphragm 21 and the rim portion 22 are provided.
The first strain sensor 31 and the second strain sensor 32 each have a common structure. Accordingly, in the following description of the structure of the strain sensor 3, the first strain sensor 31 and the second strain sensor 32 are described as the strain sensor 3 without distinguishing between the first strain sensor 31 and the second strain sensor 32.

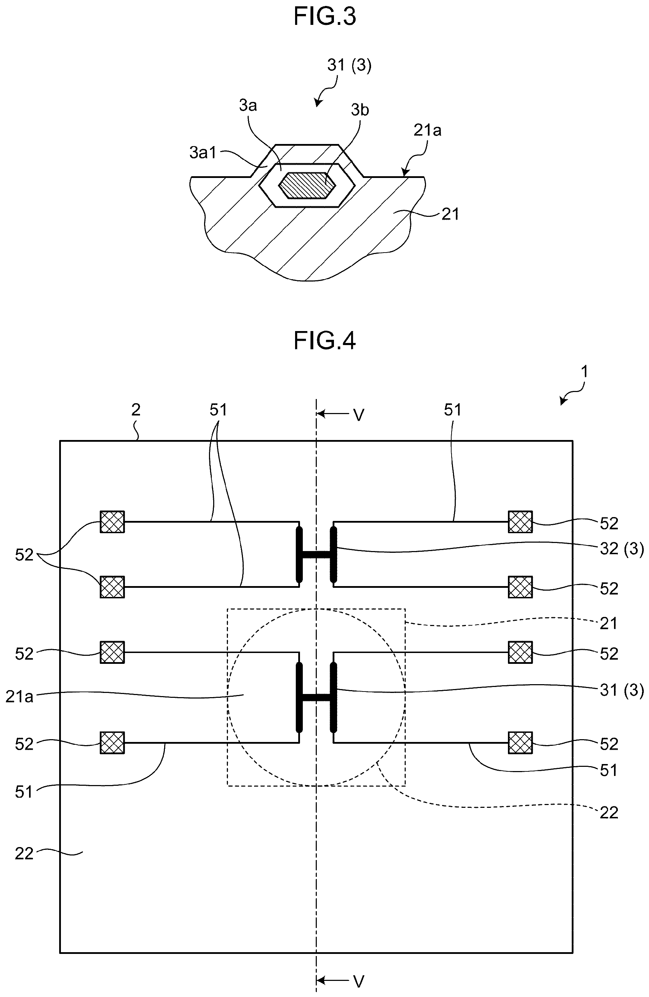

FIG. 3 is a partial enlarged cross-sectional view of a portion A illustrated in FIG. 2 in an enlarged manner. The strain sensor 3 includes a vacuum chamber 3a and a resonator 3b. The vacuum chamber 3a is formed on the first surface 21a of the silicon substrate 2. The vacuum chamber 3a is a space that is surrounded by a cavity 3a1 that is formed on the first surface 21a of the silicon substrate 2. The interior of the vacuum chamber 3a is a vacuum.
The resonator 3b is accommodated in the vacuum chamber 3a. As illustrated in FIG. 2, the strain sensor 3 is formed in an H-shape in a plan view, and both of the resonator 3b and the vacuum chamber 3a are also formed in the H-shape. The end portions of the resonator 3b are fixed to the silicon substrate 2. A gap is formed between the resonator 3b and an inner wall surface of the vacuum chamber 3a except for the fixed both ends of the resonator 3b. A resonance frequency of the resonator 3b changes due to a strain occurring in the resonator 3b.
On the silicon substrate 2, a wiring line 51 that is connected to the resonator 3b is provided. A terminal 52 to which an external wiring line, such as a bonding wire, is connected is provided at the end portion of the wiring line 51. Furthermore, the shape of the strain sensor 3 included in the chemical sensor 1 is not limited to the exemplified H-shape as long as the strain sensor 3 has a configuration in which the resonator 3b is accommodated in the vacuum chamber 3a. For example, the strain sensor 3 may also be formed in an I-shape. Furthermore, the number of and the position of the wiring lines 51 and the terminals 52 formed on the silicon substrate 2 may be changed in accordance with the shape or the like of the strain sensor 3 as appropriate.

Regarding adsorption membrane

The adsorption membrane 4 is provided on the second surface 21b of the diaphragm 21. The volume of the adsorption membrane 4 changes by adsorbing a specific substance. An example of the specific substance that the adsorption membrane 4 adsorbs includes hydrogen.
The adsorption membrane 4 that adsorbs hydrogen is formed by a material that easily adsorbs and release hydrogen and whose volume changes in accordance with the adsorption and the release of hydrogen. Examples of this kind of material include palladium, magnesium, titanium, vanadium, zirconium, lanthanum, iron, nickel, or tantalum, or include an alloy that includes one of these metals. Moreover, as the material that is used for the adsorption membrane 4, an amorphous material may also be chosen, instead of a material having a crystalline property, in order to enhance the property of absorption and release of hydrogen. Furthermore, by applying an alloy to the material that is used for the adsorption membrane 4, it is possible to improve resistance to hydrogen embrittlement of the adsorption membrane 4.
The specific substance that the adsorption membrane 4 adsorbs is not limited to hydrogen. For example, examples of the specific substance that the adsorption membrane 4 adsorbs include carbon monoxide and carbon dioxide. For example, an example of the material that is used for the adsorption membrane 4 that adsorbs carbon dioxide includes lithium composite oxide, such as lithium zirconate. Moreover, in addition to the specific substance that is in the gaseous state, the specific substance that has been dissolved in a liquid and becomes molecules and ions are also adsorbed by the adsorption membrane 4.

Regarding concentration measurement of specific substance performed by using chemical sensor

The chemical sensor 1 is able to measure a concentration of the specific substance that is included in the medium that is brought into contact with the adsorption membrane 4.

The volume of the adsorption membrane 4 increases as a result of adsorbing the specific substance. A description will be given here by referring back to FIG. 2. As a result of an increase in the volume of the adsorption membrane 4, a stress is applied to a tensile direction on the second surface 21b of the diaphragm 21, and accordingly, a tensile strain occurs. At this time, a stress in a compression direction is applied onto the first surface 21a that corresponds to the rear surface of the second surface 21b, and a compressive strain occurs. The first strain sensor 31 is provided on the first surface 21a of the diaphragm 21. As a result of the compressive strain occurring on the first surface 21a, a compressive strain also occurs in the resonator 3b that is included in the first strain sensor 31. As a result of the compressive strain occurring, the resonance frequency of the resonator 3b changes.

Moreover, at the boundary portion between the diaphragm 21 and the rim portion 22 on the first surface 21a side of the silicon substrate 2, a tensile strain occurs as a result of a stress being applied in the tensile direction. The second strain sensor 32 is provided at the boundary portion between the diaphragm 21 and the rim portion 22. As a result of the tensile strain occurring at the boundary portion between the diaphragm 21 and the rim portion 22, a tensile strain also occurs in the resonator 3b that is included in the second strain sensor 32. As a result of the tensile strain occurring, the resonance frequency of the resonator 3b changes.

Here, a change in the volume of the adsorption membrane 4 increases as the amount of the specific substance that the adsorption membrane 4 has adsorbed is large, and accordingly, the strain occurring in the resonator 3b also becomes larger. Moreover, when the strain occurring in the resonator 3b becomes larger, a change in resonance frequency also becomes greater. The amount of the specific substance adsorbed by the adsorption membrane 4 is proportional to the amount of the specific substance included in the medium that is brought into contact with the adsorption membrane 4. Therefore, in the chemical sensor 1, it is possible to measure the concentration of the specific substance included in the medium by measuring the resonance frequency of the resonator 3b. Furthermore, from the viewpoint of measuring the concentration with high accuracy, it is preferable that the resonator 3b that is included in the first strain sensor 31 and the resonator 3b that is included in the second strain sensor 32 do not match within the range of the respective resonance frequencies that are changing each other. Furthermore, it is also possible to measure the concentration of the specific substance by arranging only the first strain sensor 31. Moreover, if the adsorption membrane 4 is formed thicker, it is possible to increase the amount of the adsorbable specific substance. Therefore, even in an environment in which the concentration of the specific substance is high, it is also possible to perform concentration measurement. On the other hand, if the adsorption membrane 4 is formed thicker, an adsorption speed and a release speed of the specific substance decrease, a responsiveness of the concentration measurement may possibly be degraded. Therefore, it is desirable that the adsorption membrane 4 is formed thinner as much as possible within the range in which the concentration measurement of the specific substance is able to be performed.

In the chemical sensor 1 according to the first embodiment, the resonator 3b is provided in an interior of the vacuum chamber 3a that is covered by a cavity 3a1. Therefore, the resonator 3b is not in contact with the medium whose concentration is measured by the chemical sensor 1. As a result of this, an influence of a change in the resonance frequency of the resonator 3b is not affected by a state of the medium, that is, whether the medium is in a gaseous or liquid state. Therefore, in the chemical sensor 1, it is possible to stably and accurately measure the concentration of the specific substance contained in the medium even when the state of the medium varies.

Regarding pressure measurement performed by using chemical sensor

In the chemical sensor 1, a strain also occurs in the resonator 3b by the pressure (the static pressure) received from the medium. The strain that occurs in the resonator 3b is proportional to the static pressure received from the medium. Therefore, in the chemical sensor 1, by measuring the resonance frequency of the resonator 3b, it is possible to measure the static pressure of the medium.

Regarding influence of adsorption amount of specific substance onto adsorption membrane

An adsorption amount of the specific substance with respect to the adsorption membrane 4 changes in accordance with the concentration of the specific substance, but, in addition to this, the adsorption amount of the specific substance with respect to the adsorption membrane 4 is also affected by fluctuations in pressure and temperature. In the chemical sensor 1, as described above, it is possible to measure the pressure of the medium, so that it is possible to calibrate the measurement value of the concentration on the basis of the measured pressure.

Moreover, a diode is formed between the silicon substrate 2 made of silicon and the wiring line 51. As a result of this, the resistance value of the diode changes due to the temperature. As a result of this, by measuring the resistance value of the silicon substrate 2, it is possible to measure the temperature. Therefore, it is possible to measure the temperature by the chemical sensor 1 by itself and calibrate the measurement value of the concentration on the basis of the measured temperature, without providing a thermometer or the like in order to measure the temperature.

In this way, in the chemical sensor 1, it is possible to perform the concentration measurement with higher accuracy by calibrating the measurement value of the concentration by measuring the pressure and the temperature.

Regarding first modification

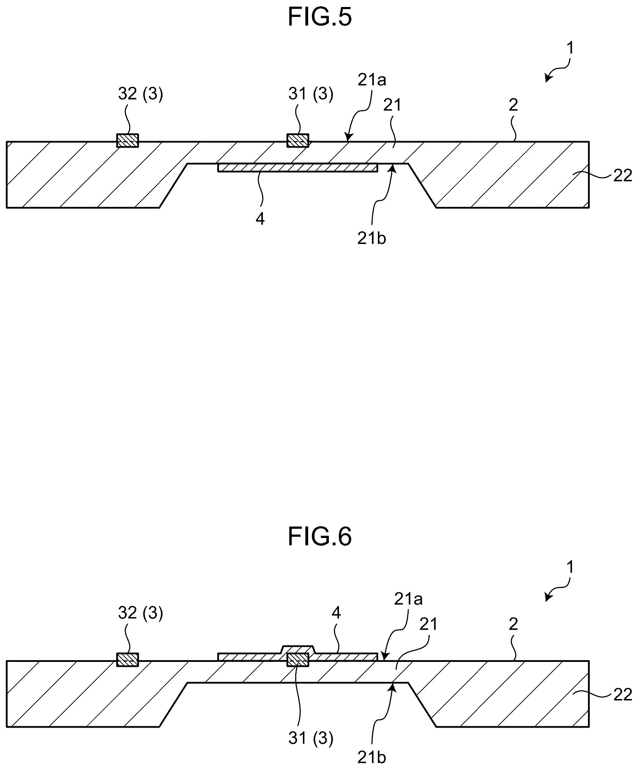

FIG. 4 is a plan view of a chemical sensor according to a first modification. FIG. 5 is a cross-sectional view of the chemical sensor taken along line V-V illustrated in FIG. 4. In the chemical sensor 1 according to the first modification, the second strain sensor 32 is provided at a position at which the second strain sensor 32 overlaps with the rim portion 22 by avoiding the position of the diaphragm 21 in a plan view. In the resonator 3b included in the second strain sensor 32 that is provided at the position at which the second strain sensor 32 overlaps with the rim portion 22 by avoiding the position of the diaphragm 21, a strain caused by a deformation of the diaphragm 21 does not occur. In other words, even when the diaphragm 21 is deformed caused by a change in volume of the adsorption membrane 4, a strain does not occur in the resonator 3b included in the second strain sensor 32. In this way, it is possible to limit the strain that occurs in the resonator 3b included in the second strain sensor 32 to the compressive strain that occurs due to the static pressure applied from the medium. Therefore, by measuring the resonance frequency of the resonator 3b included in the second strain sensor 32, it is possible to further accurately measure the static pressure applied to the medium.

Regarding second modification

FIG. 6 is a cross-sectional view of a chemical sensor according to a second modification. The cross section illustrated in FIG. 6 corresponds to the cross sections illustrated in FIG. 2 and FIG. 5. In the chemical sensor 1 according to the second modification, the adsorption membrane 4 is provided on the first surface 21a of the diaphragm 21. As a result of this, the first strain sensor 31 is covered by the adsorption membrane 4.

If the volume of the adsorption membrane 4 increases as a result of the adsorption membrane 4 provided on the first surface 21a adsorbing the specific substance, a tensile strain occurs as a result of a stress being applied to the first surface 21a of the diaphragm 21 in the tensile direction. Because of this, a tensile strain also occurs in the resonator 3b included in the first strain sensor 31. Therefore, it is also possible to measure the concentration of the specific substance even in a case of the configuration in which the adsorption membrane 4 is provided on the first surface 21a.

As illustrated in FIG. 6, similarly to the first modification, in a case where the second strain sensor 32 is formed at the rim portion 22 by avoiding the diaphragm 21, the strain caused by a deformation of the diaphragm 21 does not occur in the resonator 3b included in the second strain sensor 32.

Here, in a case where the chemical sensor 1 is used in an environment in which a high pressure is applied to the medium, there is a need to prevent buckling of the resonator 3b. In a case where the initial tensile force applied to the resonator 3b is denoted by $\varepsilon_0$, the compressive strain that occurs due to the static pressure of the medium is denoted by $\varepsilon_p$, and the compressive strain that occurs due to the deformation of the diaphragm 21 is denoted by $\varepsilon_d$, the condition that buckling occurs in the resonator 3b is represented by mathematical formula (1) described below.

$$\text{buckling strain of the resonator } 3b < \varepsilon_0 - \varepsilon_p - \varepsilon_d \qquad (1)$$

In the chemical sensor 1 according to the second modification, the strain that occurs in the resonator 3b included in the first strain sensor 31 caused by the deformation of the diaphragm 21 is the tensile strain and is not the compressive strain. Moreover, the strain does not occur, due to the deformation of the diaphragm 21, in the resonator 3b included in the second strain sensor 32. As a result of this, in both of the resonator 3b included in the first strain sensor 31 and the resonator 3b included in the second strain sensor 32, $\varepsilon_d$ that occurs caused by the deformation of the diaphragm 21 becomes zero in the above described mathematical formula (1), it is possible to secure a larger range of $\varepsilon_p$ that is the range in which $\varepsilon_0 - \varepsilon_p - \varepsilon_d$ does not exceed the buckling strain. Therefore, this makes it possible to set the measurable pressure range more broadly by the chemical sensor 1.

Regarding formation of permeation prevention membrane

In the chemical sensor 1 as exemplified as above, it may be possible to form a permeation prevention membrane (not illustrated) that prevents permeation of the specific substance so as to cover at least one of the first surface 21a and the second surface 21b of the silicon substrate 2. In an area in which the adsorption membrane 4 is provided, the permeation prevention membrane is formed between the adsorption membrane 4 and the diaphragm 21.

The permeation prevention membrane is, for example, a hydrogen permeation prevention membrane that prevents permeation of hydrogen. As a result of the hydrogen permeation prevention membrane being formed, it is possible to prevent hydrogen from generating a harmful effect on the strain sensor 3 by permeating the silicon substrate 2. The hydrogen permeation prevention membrane is desirably formed by a material having characteristics in that a diffusion coefficient of hydrogen is low, a membrane adhesion is good, a membrane stress applied to the diaphragm 21 after the membrane has been formed is small, membrane defects are few, and the like. An example of the membrane formed by using the material that satisfies the above described conditions includes a membrane made of gold, a ceramic membrane, such as a nitride membrane or an alumina membrane, or the like.

The permeation prevention membrane is able to prevent a harmful effect on the strain sensor 3 as a result of hydrogen contained in the medium permeating the silicon substrate 2 from the surface as long as permeation prevention membrane is formed on at least one of the first surface 21a and the second surface 21b.

Regarding installation example of chemical sensor

The chemical sensor 1 that has been exemplified up to here is constituted such that the same medium is in contact with the first surface 21a and the second surface 21b, and a deformation of the diaphragm 21 is not observed caused by the pressure applied from the medium, so that the deformation of the diaphragm 21 is used for a measurement of the concentration of the specific substance. On the other hand, the chemical sensor 1 may be installed a measurement apparatus that is constituted such that a different pressure is applied to each of the first surface 21a and the second surface 21b of the diaphragm 21. In the measurement apparatus, the diaphragm 21 is deformed caused by a difference between the pressure applied to the first surface 21a and the pressure applied to the second surface 21b. In the measurement apparatus, by using the deformation of the diaphragm, a differential pressure that is the difference between the pressure applied to the first surface 21a and the pressure applied to the second surface 21b is measured. This type of measurement apparatus is also referred to as a differential pressure sensor.

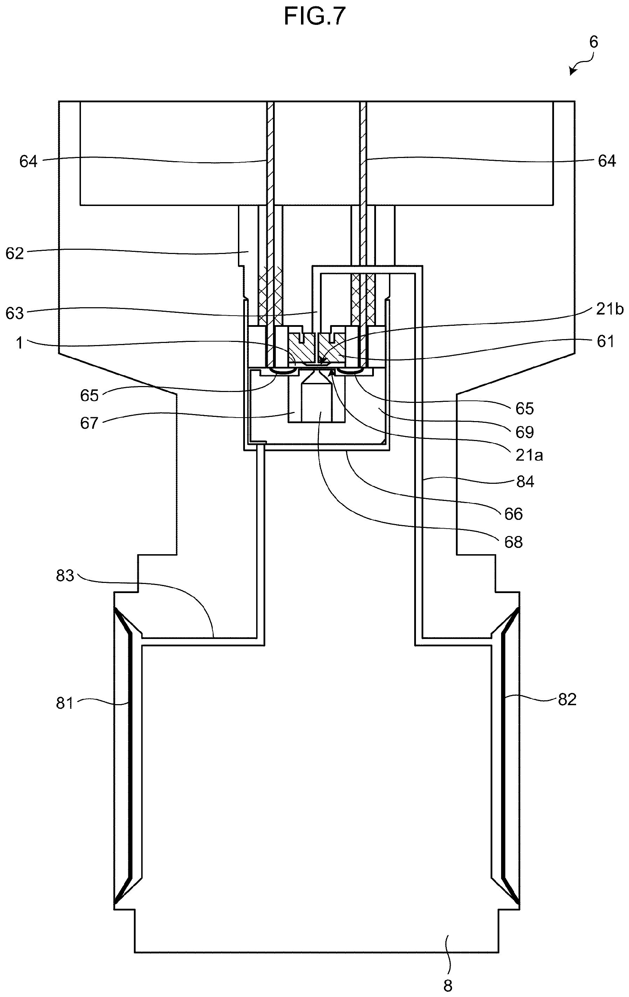

FIG. 7 is a cross-sectional view illustrating one example of the measurement apparatus on which the chemical sensor according to the first embodiment is mounted. A measurement apparatus 6 is a differential pressure sensor described above.

In the measurement apparatus 6, the chemical sensor 1, a base 61, a pedestal base 62, a cap 66, a yoke 67, a magnet 68, and a magnet holder 69 are accommodated in the interior of a housing 8.

In the measurement apparatus 6, the chemical sensor 1 is fixed to the base 61. The base 61 is fixed to the pedestal base 62. The rim portion 22 (see also FIG. 2, etc.) included in the chemical sensor 1 abuts against the base 61. The rim portion 22 is formed so as to surround the circumference of the diaphragm 21, so that the space between the second surface 21b and the base 61 and the space from which the first surface 21a is exposed are partitioned by the rim portion 22. Oil that corresponds to the medium is filled in a gap between the second surface 21b and the base 61. The oil that is filled in the gap between the second surface 21b and the base 61 comes into contact with the second surface 21b of the diaphragm 21. A passage 63 that communicates with the space from which the second surface 21b is exposed is formed on the pedestal base 62 and the base 61.

The measurement apparatus 6 includes a hermetic terminal 64 that passes through the pedestal base 62 and that protrudes at a position avoiding the position of the base 61. The hermetic terminal 64 is electrically connected to the terminal 52 (see also FIG. 1, etc.) that is formed on the first surface 21a of the chemical sensor 1 via a bonding wire 65. An electrical signal that indicates the resonance frequency of the resonator 3b is output from the chemical sensor 1. The electrical signal that has been output from the chemical sensor 1 is transmitted to an outside of the measurement apparatus 6 via the bonding wire 65 and the hermetic terminal 64.

The cap 66 is attached on the pedestal base 62. By attaching the cap 66, a space in which the chemical sensor 1 and the base 61 is accommodated is formed by both of the cap 66 and the pedestal base 62. The yoke 67, the magnet 68, and the magnet holder 69 are provided in the space in which the chemical sensor 1 and the base 61 are accommodated. Both of the yoke 67 and the magnet 68 are held by the magnet holder 69. Both of the yoke 67 and the magnet 68 are held at a position that is opposite the first surface 21a of the silicon substrate 2.

Oil that is the medium is filled between the yoke 67 and the magnet 68 and the first surface 21a of the silicon substrate 2. The oil that is filled between the yoke 67 and the magnet 68 and the first surface 21a of the silicon substrate 2 comes into contact with the first surface 21a of the diaphragm 21.

Each of the yoke 67 and the magnet 68 that is disposed opposite the first surface 21a of the diaphragm 21 functions as an excitation portion that causes the resonator 3b included in the strain sensor 3 to excite. In this way, a drive method of the chemical sensor 1 that causes the resonator 3b to excite by using the excitation portion that includes both of the yoke 67 and the magnet 68 is also referred to as an electromagnetic drive method. For the chemical sensor 1 that uses the electromagnetic drive method illustrated in FIG. 7, the chemical sensor 1 that includes the strain sensor 3 that is formed in the H-shaped type illustrated in FIG. 1 and FIG. 4 is used.

In the measurement apparatus 6 that uses the electromagnetic drive method, in some cases, a gap between the first surface 21a of the silicon substrate 2 and the yoke 67 is narrow, that is, about 10 μm, so that a path through which the specific substance enters the first surface 21a is complicated. In a case where a concentration of the specific substance is measured by the adsorption membrane 4 that is provided on the first surface 21a, responsiveness tends to decrease. As a result of this, in the chemical sensor 1 that uses the electromagnetic drive method, in some cases, there may preferably be a case in which the adsorption membrane 4 is formed on the second surface 21b and the concentration of the medium that comes into contact with the second surface 21b is measured.

On the other hand, with the chemical sensor 1 that includes the strain sensor 3 that is formed in an I-shaped type, a structure that causes the resonator 3b to excite is formed in the chemical sensor 1 itself. In this way, the drive method for causing the resonator 3b to excite by using the structure that is formed in the chemical sensor 1 itself is also referred to as an electrostatic drive method. In a case where the chemical sensor 1 that uses the electrostatic drive method is used by the measurement apparatus 6, there is no need to provide the yoke 67 and the magnet 68 that cause the resonator 3b to excite. As a result of this, as compared with a case of the electromagnetic drive method, it is possible to secure a larger space in which the medium is filled in the circumference of the first surface 21a on which the strain sensor 3 is formed. As a result of this, the specific substance easily enters the first surface 21a. Therefore, in the chemical sensor 1 that uses the electrostatic drive method, in some cases, there may preferably be a case in which the adsorption membrane 4 is formed on the first surface 21a and the concentration of the medium that comes into contact with the first surface 21a is measured.

In the housing 8, both of a first pressure sensing diaphragm 81 and a second pressure sensing diaphragm 82 are formed. In the measurement apparatus 6, the medium that comes into contact with both of the first pressure sensing diaphragm 81 and the second pressure sensing diaphragm 82 from outside of the housing 8 is the medium whose differential pressure is measured by the measurement apparatus 6. A space is formed an inner side of each of the first pressure sensing diaphragm 81 and the second pressure sensing diaphragm 82, so that the first pressure sensing diaphragm 81 and the second pressure sensing diaphragm 82 are formed into a thin membrane, and are deformed by the pressure received from the medium.

The space that is formed on the inner side of the first pressure sensing diaphragm 81 is joined to the space from which the first surface 21a of the chemical sensor 1 is exposed by passing through a first housing passage 83 that is formed in the inner side of the housing 8. Therefore, in the measurement apparatus 6, oil is filled in the space located in the inner side of the first pressure sensing diaphragm 81 and in the first housing passage 83, similarly to the space from which the first surface 21a is exposed.

The space that is formed in the inner side of the second pressure sensing diaphragm 82 is joined to the space from which the second surface 21b of the chemical sensor 1 is exposed by passing through a second housing passage 84 and the passage 63 that are formed in the inner side of the housing 8. Therefore, in the measurement apparatus 6, oil is filled in the space located in the inner side of the second pressure sensing diaphragm 82, the second housing passage 84, and in the passage 63, similarly to the space from which the second surface 21b is exposed.

The space from which the first surface 21a is exposed and the space from which the second surface 21b is exposed are partitioned, and do not communicate with each other. As a result of this, in a case where the pressure that is received by the first pressure sensing diaphragm 81 from the medium is different from the pressure that is received by the second pressure sensing diaphragm 82 from the medium, the pressure that is received by the first surface 21a is different from the pressure that is received by the second surface 21b, so that the diaphragm 21 included in the chemical sensor 1 is deformed. By detecting the deformation of the diaphragm 21 by the strain sensor 3, it is possible to measure a differential pressure that is a difference between the pressure received by the first pressure sensing diaphragm 81 from the medium and the pressure received by the second pressure sensing diaphragm 82 from the medium.

On the other hand, in the measurement apparatus 6, as a result of the adsorption membrane 4 being provided on the first surface 21a or the second surface 21b of the diaphragm 21 included in the chemical sensor 1, it is also possible to measure the concentration of the specific substance of the medium that is in contact with that surface. In the adsorption membrane 4, not only the specific substance that is in the gaseous state but also a molecule corresponding to the specific substance or an ion corresponding the specific substance that is dissolved in a liquid, such as oil, are adsorbed. As a result of this, in also a case in which oil is in contact with the adsorption membrane 4 as in a case of the measurement apparatus 6, it is possible to measure the concentration of the specific substance.

Moreover, as described above, in the chemical sensor 1, the resonator 3b is provided in the interior of the vacuum chamber 3a that is covered by the cavity 3a1, so that it is also possible to stably measure with high accuracy the concentration of the specific substance that is contained in the medium even when the state of the medium varies. As a result of this, this makes it also possible to stably measure a concentration of the specific substance with respect to the medium in which both of a liquid and gas exist together. As a result of this, even in a case where air bubbles occur in a liquid medium, it is possible to stably measure the concentration. For example, air bubbles occur in a case where some of liquid medium exhibits a change in state, or in a case in which a substance dissolving in the liquid medium exceeds its dissolvable amount.

In the measurement apparatus 6, by switching an operation in a mode (differential pressure measurement mode) for measuring a differential pressure of the medium that is in contact with both of the first pressure sensing diaphragm 81 and the second pressure sensing diaphragm 82, and an operation in a mode (concentration measurement mode) for measuring a concentration of the specific substance of the medium (oil) that is in contact with the surface on which the adsorption membrane 4 is provided, it is possible to measure the differential pressure and the concentration of the specific substance. The processes performed in the arithmetic unit that processes the signal acquired from the chemical sensor 1 are sometimes different between in the differential pressure measurement mode and in the concentration measurement mode.

In the description below, it is assumed that the specific substance is hydrogen. In some cases, hydrogen that has permeated through the first pressure sensing diaphragm 81 or the second pressure sensing diaphragm 82 comes to be mixed in the oil that is filled in the interior of the housing 8. In a case where hydrogen comes to be mixed in the oil, hydrogen is dissolved in the oil up to a dissolvable amount, but, if the dissolvable amount exceeds, air bubbles of hydrogen occur. In a case where the mixed hydrogen is able to be dissolved in the oil, there will be no problems with the measurement of the differential pressure. However, if hydrogen exceeds its dissolvable amount and air bubbles occur, the pressure received by the first pressure sensing diaphragm 81 or the second pressure sensing diaphragm 82 is not correctly transmitted to the diaphragm 21 included in the chemical sensor 1. In other words, there may be a case in which a problem that the pressure measurement suddenly become impossible caused by an occurrence of the air bubbles in the oil.

Here, if hydrogen comes to be mixed in the oil during the operation performed in the differential pressure measurement mode, the hydrogen is adsorbed into the adsorption membrane 4, and the volume of the adsorption membrane 4 changes. The diaphragm 21 is deformed as a result of a change in volume of the adsorption membrane 4, abnormality occurs in the measurement value of the differential pressure. By measuring the hydrogen concentration by switching the operation to the concentration measurement mode at the time at which this abnormal value has been detected, it is possible to predict that the differential pressure measurement will become impossible due to an occurrence of air bubbles.

To detect the hydrogen mixing into the oil, two cases, that is, a case in which a single unit of the measurement apparatus 6 is used and a case in which two units of the measurement apparatuses 6 are used, are conceivable. First, the procedure of the measurement of the hydrogen concentration performed by using the single unit of the measurement apparatus 6.

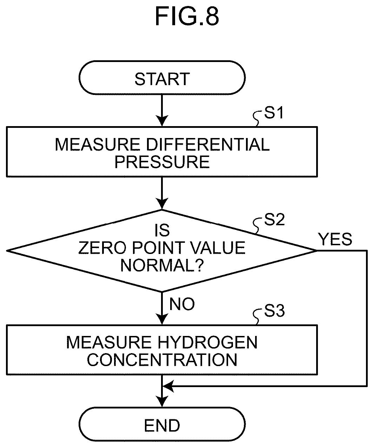

FIG. 8 is a flowchart illustrating the measurement procedure of the hydrogen concentration performed by using the single unit of the measurement apparatus. First, the measurement apparatus 6 measures the differential pressure by operating in the differential pressure measurement mode (Step S1). Specifically, the differential pressure is calculated on the basis of the resonance frequency of the resonator 3b included in each of the first strain sensor 31 and the second strain sensor 32, and on the basis of the resistance value of the silicon substrate 2.

Then, a checking of a zero point value of the calculated differential pressure is performed (Step S2). If the zero point value of the differential pressure is normal (Yes at Step S2), the operation in the differential pressure measurement mode without any change.

On the other hand, if the zero point value of the differential pressure is abnormal (No at Step S2), the operation of the measurement apparatus 6 is switched to the operation in the concentration measurement mode, and the hydrogen concentration is measured (Step S3). In also the operation of the concentration measurement mode, similarly to the operation in the differential pressure measurement mode, the hydrogen concentration of the oil is calculated on the basis of the resonance frequency of the resonator 3b and the resistance value of the silicon substrate 2.

In a case where the calculated hydrogen concentration is close to the dissolvable amount of the hydrogen into the oil, it is predicted that air bubbles occur in the oil in a short time and a problem that the differential pressure measurement will become impossible occurs. In this way, by installing the chemical sensor 1 in the measurement apparatus 6, it is possible to predict an occurrence of the problem caused by hydrogen coming to be mixed, and it is possible to take action in advance. Furthermore, in also a case in which the differential pressure becomes impossible to measure, by calculating the hydrogen concentration, it is possible to determine whether or not the cause of inability to measure the differential pressure is a state in which hydrogen comes to be mixed.

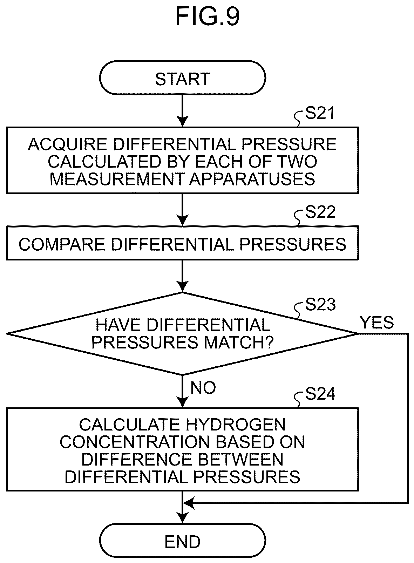

In the following, the measurement procedure of the hydrogen concentration performed by using the two units of the measurement apparatuses 6 will be described. FIG. 9 is a flowchart illustrating the measurement procedure of the hydrogen concentration performed by using the two units of the measurement apparatuses.

First, the differential pressure that has been calculated by each of the two measurement apparatuses 6 is acquired (Step S21). Furthermore, the calculation of the differential pressure performed in each of the measurement apparatuses 6 is performed by the same process as that performed at Step S1 illustrated in FIG. 9. Then, the differential pressures that have been calculated by the respective two measurement apparatuses 6 are compared (Step S22). If the differential pressures that have been calculated by the respective two measurement apparatuses 6 match (Yes at Step S23), the operation in the differential pressure measurement mode is continued without any change.

On the other hand, if there is an error in the differential pressure calculated by each of the two measurement apparatuses 6 (No at Step S23), there is a possibility that hydrogen has been adsorbed into the adsorption membrane 4 included in one of the measurement apparatuses 6, so that the operation of each of the measurement apparatuses 6 is switched to the operation in the concentration measurement mode.

In the operation performed in the concentration measurement mode, the hydrogen concentration is calculated on the basis of the difference between the differential pressures that are calculated by the two measurement apparatuses 6 (Step S24).

In a case where the calculated hydrogen concentration is close to the dissolvable amount of the hydrogen into the oil, it is predicted that air bubbles occur in the oil in a short time and a problem that the differential pressure measurement will become impossible occurs. In this way, by installing the chemical sensor 1 in the measurement apparatus 6, it is possible to predict an occurrence of the problem caused by hydrogen coming to be mixed, and it is possible to take action in advance. Furthermore, in also a case in which the differential pressure becomes impossible to measure, by calculating the hydrogen concentration, it is possible to determine whether or not the cause of inability to measure the differential pressure is a state in which hydrogen comes to be mixed.

Furthermore, the adsorption membrane 4 may also be formed on both of the first surface 21a and the second surface 21b. In the measurement apparatus 6, as described above, the space from which the first surface 21a of the silicon substrate 2 is exposed and the space in which the second surface 21b is exposed are partitioned. In other words, the medium that comes into contact with the first surface 21a and the medium that comes into contact with the second surface 21b are different. If the adsorption membrane 4 is formed on both of the first surface 21a and the second surface 21b, even in a case where the hydrogen comes to be mixed into one of the mediums, it is also possible to perform the concentration measurement on the basis of a change in volume of the adsorption membrane 4.

Others

Some examples of combinations of the disclosed technical features will be described below.

(1) A chemical sensor comprising: a silicon substrate on which a diaphragm is formed; a strain sensor that is provided on the silicon substrate; and an adsorption membrane that is provided at the diaphragm, and whose volume changes by adsorbing a specific substance, wherein the strain sensor includes a vacuum chamber that is formed in the diaphragm, and a resonator that is formed in an interior of the vacuum chamber.

(2) The chemical sensor according to (1), wherein the diaphragm includes a first surface on which the strain sensor is provided, and a second surface that is a rear surface of the first surface, and the adsorption membrane is provided on the second surface.

(3) The chemical sensor according to (1) or (2), wherein a drive method for causing the resonator to excite is an electromagnetic drive method.

(4) The chemical sensor according to any one of (1) to (3), further comprising a permeation prevention membrane that is provided on the second surface, and that prevents permeation of the specific substance by covering the second surface.

(5) The chemical sensor according to any one of (1) to (4), wherein the permeation prevention membrane is also provided on the first surface and prevents the permeation of the specific substance.

(6) The chemical sensor according to (1), wherein the diaphragm includes a first surface on which the strain sensor is provided, and a second surface that is a rear surface of the first surface, and the adsorption membrane is provided on the first surface.

(7) The chemical sensor according to any one of (1) to (6), wherein a drive method for causing the resonator to excite is an electrostatic drive method.

(8) The chemical sensor according to (6) or (7), further comprising a permeation prevention membrane that is provided on the first surface, and that prevents permeation of the specific substance by covering the first surface.

(9) The chemical sensor according to any one of (6) to (8), wherein the permeation prevention membrane is also provided on the second surface and prevents the permeation of the specific substance.

(10) The chemical sensor according to any one of (1) to (9), wherein the specific substance adsorbed by the adsorption membrane is hydrogen.

(11) A measurement apparatus comprising: a chemical sensor according to any one of (2) to (9); and a housing that accommodates the chemical sensor in an interior of the housing, wherein a first pressure sensing diaphragm, a second pressure sensing diaphragm, a first housing passage that communicates between an inner side of the first pressure sensing diaphragm and a space from which the first surface is exposed, and a second housing passage that communicates between an inner side of the second pressure sensing diaphragm and a space from which the second surface is exposed are formed in the housing, and oil is filled in the space from which the first surface is exposed, the first housing passage, the space from which the second surface is exposed, and the second housing passage.

According to the present disclosure, an advantage is provided in that it is possible to obtain a chemical sensor capable of stably and accurately measuring a concentration of a specific substance contained in a medium.

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

**Claims**

1. A chemical sensor (1) comprising:

   a silicon substrate (2) on which a diaphragm (21) is formed;
   a strain sensor (3) that is provided on the silicon substrate; and
   an adsorption membrane (4) that is provided at the diaphragm, and whose volume changes by adsorbing a specific substance, wherein
   the strain sensor includes

      a vacuum chamber (3a) that is formed in the diaphragm, and
      a resonator (3b) that is formed in an interior of the vacuum chamber.

2. The chemical sensor according to claim 1, wherein

   the diaphragm includes

      a first surface (21a) on which the strain sensor is provided, and
      a second surface (21b) that is a rear surface of the first surface, and

   the adsorption membrane is provided on the second surface.

3. The chemical sensor according to claims 1 or 2, wherein a drive method for causing the resonator to excite is an electromagnetic drive method.

4. The chemical sensor according to any one of claims 1 to 3, further comprising a permeation prevention membrane that is provided on the second surface, and that prevents permeation of the specific substance by covering the second surface.

5. The chemical sensor according to any one of claims 1 to 4, wherein the permeation prevention membrane is also provided on the first surface and prevents the permeation of the specific substance.

6. The chemical sensor according to claim 1, wherein

   the diaphragm includes

      a first surface on which the strain sensor is provided, and
      a second surface that is a rear surface of the first surface, and

   the adsorption membrane is provided on the first surface.

7. The chemical sensor according to any one of claims 1 to 6, wherein a drive method for causing the resonator to excite is an electrostatic drive method.

8. The chemical sensor according to claims 6 or 7, further comprising a permeation prevention membrane that is provided on the first surface, and that prevents permeation of the specific substance by covering the first surface.

9. The chemical sensor according to any one of claims 6 to 8, wherein the permeation prevention membrane is also provided on the second surface and prevents the permeation of the specific substance.

10. The chemical sensor according to any one of claims 1 to 9, wherein the specific substance adsorbed by the adsorption membrane is hydrogen.

11. A measurement apparatus comprising:

a chemical sensor according to any one of claims 2 to 9; and
a housing (8) that accommodates the chemical sensor in an interior of the housing, wherein
a first pressure sensing diaphragm (81), a second pressure sensing diaphragm (82), a first housing passage (83) that communicates between an inner side of the first pressure sensing diaphragm and a space from which the first surface is exposed, and a second housing passage (84) that communicates between an inner side of the second pressure sensing diaphragm and a space from which the second surface is exposed are formed in the housing, and
oil is filled in the space from which the first surface is exposed, the first housing passage, the space from which the second surface is exposed, and the second housing passage.

# FIG.1

# FIG.2

# FIG.3

# FIG.4

# FIG.5

# FIG.6

# FIG.7

# FIG.8

```
          ┌─────────────────┐
          │      START       │
          └─────────────────┘
                   │
                   ▼              ⟋S1
     ┌──────────────────────────────┐
     │   MEASURE DIFFERENTIAL        │
     │        PRESSURE              │
     └──────────────────────────────┘
                   │
                   ▼                    ⟋S2
              ╱─────────────╲              YES
          ╱         IS         ╲──────────────┐
         ⟨   ZERO POINT VALUE    ⟩             │
          ╲      NORMAL?      ╱               │
              ╲─────────────╱                 │
                   │ NO                        │
                   ▼              ⟋S3          │
     ┌──────────────────────────────┐         │
     │   MEASURE HYDROGEN           │         │
     │     CONCENTRATION           │         │
     └──────────────────────────────┘         │
                   │◄──────────────────────────┘
                   ▼
          ┌─────────────────┐
          │       END        │
          └─────────────────┘
```

# FIG.9

```
        ┌─────────────────┐
        │      START      │
        └─────────────────┘
                 │
                 ▼              ⌐S21
┌───────────────────────────────────┐
│   ACQUIRE DIFFERENTIAL PRESSURE    │
│     CALCULATED BY EACH OF TWO      │
│      MEASUREMENT APPARATUSES       │
└───────────────────────────────────┘
                 │
                 ▼              ⌐S22
┌───────────────────────────────────┐
│   COMPARE DIFFERENTIAL PRESSURES   │
└───────────────────────────────────┘
                 │
                 ▼              ⌐S23
            ◇───────────◇              YES
        HAVE DIFFERENTIAL  ───────────────┐
        PRESSURES MATCH?                  │
            ◇───────────◇                 │
                 │ NO                      │
                 ▼              ⌐S24       │
┌───────────────────────────────────┐     │
│       CALCULATE HYDROGEN           │     │
│    CONCENTRATION BASED ON          │     │
│     DIFFERENCE BETWEEN             │     │
│    DIFFERENTIAL PRESSURES          │     │
└───────────────────────────────────┘     │
                 │◄──────────────────────┘
                 ▼
        ┌─────────────────┐
        │       END       │
        └─────────────────┘
```

## EUROPEAN SEARCH REPORT

**Application Number**

EP 25 22 4888

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/151689 A1 (THEUSS HORST [DE] ET AL) 9 May 2024 (2024-05-09) | 1,2,10 | INV.<br>G01N29/02 |
| Y | * paragraphs [0006] - [0021] *<br>* paragraphs [0039] - [0047] *<br>* figures 1-5 * | 1-11 | G01N29/036<br>G01N29/24 |
| Y | MASAYOSHI ESASHI ET AL: "Vacuum-Sealed Silicon Micromachined Pressure Sensors", PROCEEDINGS OF THE IEEE IEEE. NEW YORK., US,<br>vol. 86, no. 8, 1 August 1998 (1998-08-01)<br>, XP011044065,<br>ISSN: 0018-9219<br>* page 1635 - page 1637; figures 11-17 * | 1-10 | |
| Y | KYOICHI IKEDA ET AL: "THREE-DIMENSIONAL MICROMACHINING OF SILICON PRESSURE SENSOR INTEGRATING RESONANT STRAIN GAUGE ON DIAPHRAGM.", SENSORS AND ACTUATORS A: PHYSICAL ELSEVIER BV, NL,<br>vol. A23., no. 01 / 03.,<br>1 April 1990 (1990-04-01), pages 1007-1010., XP000355788,<br>ISSN: 0924-4247<br>* page 1010; figures 1-3 * | 1-10 | TECHNICAL FIELDS SEARCHED (IPC)<br><br>G01N |
| Y | EP 0 456 029 A1 (YOKOGAWA ELECTRIC CORP [JP]) 13 November 1991 (1991-11-13)<br>* the whole document * | 1-11 | |
| Y | JP 2002 318167 A (YAMATAKE CORP) 31 October 2002 (2002-10-31)<br>* paragraph [0004]; figure 1 * | 4,5,8,9 | |

The present search report has been drawn up for all claims

| Place of search | Date of completion of the search | Examiner |
|---|---|---|
| Munich | 19 May 2026 | Roetsch, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 22 4888

This annex lists the patent family members relating to the patent documents cited in  the above-mentioned European search report.
The members are as contained in the European Patent Office EDP file on
The European Patent Office is in no way liable for these particulars which are merely given for the purpose of information.

19-05-2026

| Patent document cited in search report | Publication date | Patent family member(s) | Publication date |
|---|---|---|---|
| US 2024151689 A1 | 09-05-2024 | CN 117990754 A<br>DE 102022211627 A1<br>US 2024151689 A1 | 07-05-2024<br>08-05-2024<br>09-05-2024 |
| EP 0456029 A1 | 13-11-1991 | DE 456029 T1<br>DE 69105809 T2<br>EP 0456029 A1<br>US 5123282 A | 11-06-1992<br>11-05-1995<br>13-11-1991<br>23-06-1992 |
| JP 2002318167 A | 31-10-2002 | NONE | |

EPO FORM P0459

For more details about this annex : see Official Journal of the European Patent Office, No. 12/82

**REFERENCES CITED IN THE DESCRIPTION**

**Patent documents cited in the description**

- JP 2024231231 A
- JP 2020019102 A